# EUROPÄISCHE PATENTSCHRIFT

(11) **EP 4 522 038 B1**
(45) Veröffentlichungstag und Bekanntmachung des Hinweises auf die Patenterteilung: **24.06.2026**
(21) Anmeldenummer: 23704373.2
(22) Anmeldetag: 16.02.2023
(51) Int. Cl.: A61B 17/115, A61B 90/00

(54) **SCHUTZABDECKUNG FÜR EIN CHIRURGISCHES INSTRUMENT**
PROTECTIVE COVER FOR A SURGICAL INSTRUMENT
HOUSSE DE PROTECTION POUR UN INSTRUMENT CHIRURGICAL

(30) Priorität: 24.02.2022 EP 22158649
(43) Veröffentlichungstag der Anmeldung: 19.03.2025
(73) Patentinhaber: IMD Tech GmbH, 88069 Tettnang (DE)
(72) Erfinder: DIETHELM, Nils, 6469 Schattdorf (CH); DIETHELM, Benjamin, 80001 Zürich (CH)
(74) Vertreter: Schaad, Balass, Menzl & Partner AG
(86) Internationale Anmeldenummer: PCT/EP2023/053878
(87) Internationale Veröffentlichungsnummer: WO 2023/161117

(56) Entgegenhaltungen:
- EP-A1- 2 730 237
- EP-A2- 2 039 316
- WO-A1-02/00121
- US-A1- 2011 248 067

## Beschreibung

Die Erfindung betrifft eine Schutzabdeckung für ein chirurgisches Instrument, insbesondere für ein Klammernahtgerät oder einen Zirkularstapler, gemäss dem Oberbegriff des Anspruch 1.

Bei den minimalinvasiven Operationsverfahren werden diverse chirurgische Instrumente verwendet, die eigens für diesen Einsatz entwickelt wurden, bspw. Klammernahtgeräte, insbesondere Zirkularstapler. Klammernahtgeräte vereinen verschiedene Funktionalitäten an einer Einsatzstelle. Klammernahtgeräte sind hauptsächlich dazu bestimmt, eine Naht zwischen Gewebeteilen eines Organs bspw. eines Darms zu erstellen. Je nach Gerätetyp können mittels eines Klammernahtgeräts ebenfalls Teile kranker oder beschädigter Organe entfernt werden, Schnitte in Organen und Geweben gemacht werden, Verbindungen zwischen Hohlorganen hergestellt oder Blutgefässe abgeklemmt werden.

Zu diesem Zweck weist das hier verwendete Klammernahtgerät ein Gegenstück auf, das vor einer eigentlichen Verbindung der beiden Gefässhälften mittels Klammerung in der gegenüberliegenden Gefässhälfte eingenäht wird. Das Gegenstück wird auch als Gegenkappe in der Fachwelt bezeichnet. Des weiteren weist das Klammernahtgerät einen axial verschieblichen Dorn auf, der eine der beiden zuvor mittels einer Längsnaht verschlossenen Gefässhälften stirnseitig durchstösst und dann in einen hohlen Schaft des Gegenstücks eingeführt wird. Durch Zurückziehen des Dorns werden die beiden Gefässhälften gegeneinander verspannt, so dass bei Auslösung des Klammernahtgeräts die Klammern eine ringförmige Naht erzeugen, die die beiden Gefässhälften verbindet.

Des weiteren weist das Klammernahtgerät ein ringförmiges Messer auf, das als Stanzwerkzeug wirken kann und nach der Herstellung der Naht die zuvor verschlossenen Gefässhälften öffnet und so das freie Lumen des Gefässes wieder herstellt.

Das Klammernahtgerät weist einen Vorderteil auf, insbesondere einen Kopfbereich, der oft eine scharfkantige Form hat, wie dies auch der Fall beim Vorderteil anderer chirurgischen Instrumente sein kann. Folglich kann sich das Einführen des chirurgischen Instruments in den Körper des Patienten, sowie dessen genaues Positionieren an der Einsatzstelle manchmal als schwierig gestalten. Darüber hinaus besteht beispielsweise beim Vordringen des Klammernahtgeräts die Gefahr, dass das umliegende Gewebe durch den Arbeitseinsatz verletzt wird, was die Genesungszeit des Patienten verlängern und insbesondere Folgeerkrankungen nach sich ziehen könnte.

WO 2007/147439 A1 offenbart eine Vorrichtung zum Einführen und Positionieren von einem chirurgischen Instrument in den Körper eines Patienten an eine Einsatzstelle, umfassend eine Aussenumhüllung. Mindestens ein Vorderteil des chirurgischen Instruments ist einsetzbar in die Aussenumhüllung, welche an der Einsatzstelle entfernbar ist. Mittels einer Zuganordnung und Perforationen an der Spitze der Aussenumhüllung kann eine Öffnung der Aussenumhüllung erzeugt werden. Die Aussenumhüllung ist in einem Stück über den Vorderteil des chirurgischen Instruments mittels der Zuganordnung nach dem Reissen der Perforationen nach hinten herüberziehbar.

DE 20 2015 003 133 U1 offenbart eine Schutzabdeckung für ein chirurgisches Instrument, insbesondere für Klammernahtgeräte, umfassend eine Aussenumhüllung, welche derart eingerichtet ist, dass sie wenigstens teilweise einen Kopfbereich des chirurgischen Instruments abdecken kann. Ferner umfasst die Schutzabdeckung eine Betätigungseinrichtung, welche mit der Aussenumhüllung kraftübertragend verbunden ist, und aus wenigstens einem Zugelement gebildet ist. Die Aussenumhüllung ist aus einem ersten Segment und einem zweiten Segment gebildet, wobei das erste Segment und das zweite Segment mit Hilfe der Betätigungseinrichtung von einer Abdeck-Position in eine Freigabe-Position überführbar sind. Das erste Segment und das zweite Segment bleiben bei der Überführung von der Abdeck-Position in die Freigabe-Position durch wenigstens ein Gelenk miteinander verbunden.

WO02/00121 offenbart einen Gleitschutz für einen Gehäusekopf medizinischer Instrumente, der aus einer zwei Abschnitte umfassenden Abschlussvorrichtung besteht. Die Abschnitte sind an ihrer äusseren Verbindungslinie mittels stegförmigen Steckbrücken miteinander verbunden und berühren sich im eingesteckten Zustand der Abschlussvorrichtung. An der Aussenseite der beiden Abschnitte sind im unteren Bereich jeweils zwei Zugeinrichtungsbefestigungseinrichtungen vorgesehen, die als versenkte Befestigungsösen ausgebildet sind. An diesen Befestigungsösen ist jeweils eine reissfeste Schnur angeknotet. Bei einem Trenn- und Entfernvorgang des Gleitschutzes vom Gehäusekopf kann der Operateur mittels einer in einem Gehäusegriff des medizinischen Instruments angebrachten Dreheinrichtung einen Verbindungsstift aus dem Inneren des Gehäusekopfs herausfahren. Eine Spitze des Verbindungsstifts gelangt in eine in der Anlagefläche der Abschlussvorrichtung ausgebildete Führungsausnehmung und drückt bei weiterem Herausdrehen die beiden Abschnitte auseinander, bis die Steckrücken der beiden Abschnitte getrennt werden. Somit zerfällt der Gleitschutz in zwei gleich ausgebildete Abschnitte, die durch ein Ziehen der Schnüre nacheinander aus dem Darmabschnitt entfernt werden.

Die oben erwähnten Vorrichtungen stellen eine Schutzabdeckung für ein chirurgisches Instrument bereit, insbesondere für ein Klammernahtgerät, welche allerdings keine Hilfe bei der Positionierung des Kopfbereiches des chirurgischen Instruments bietet. Beim Klammernahtgerät müssen nämlich das Gegenstück und der Dorn relativ zueinander zentriert werden, bevor eine ringförmige Naht durch Klammern erzeugt werden kann. Die oben erwähnten Schutzabdeckungen ermöglichen keine Feststellung des Ortes, an dem der Dorn die Gefässhälften zur Einführung ins Gegenstück durchstechen wird. Es folgt, dass mehrere Versuche nötig sein können, um die optimale relative Position des Gegenstücks und des Dorns zu finden. Erschwerend kommt noch hinzu, dass sich der Kopfbereich bei den Bewegungen des chirurgischen Instruments während der Versuche in Gewebefalten verfangen kann.

Es ist deshalb eine Aufgabe der Erfindung, eine Schutzabdeckung für ein chirurgisches Instrument, insbesondere für ein Klammernahtgerät, zur Verfügung zu stellen, welche ein genaues, sicheres Einführen sowie Positionieren des chirurgischen Instruments in den Körper des Patienten an der Einsatzstelle ermöglicht. Insbesondere soll die Schutzabdeckung dazu beitragen, die Orientierung eines aus einem Kopfbereich des Klammernahtgeräts auszufahrenden Dorns zu bestimmen, um den Kopfbereich relativ zu einem Gegenstück des chirurgischen Instruments optimal zu positionieren bzw. zu zentrieren.

Diese Aufgabe wird erfindungsgemäss durch eine Schutzabdeckung gemäss Anspruch 1 gelöst. Bevorzugte Ausführungsformen der Erfindung sind in den abhängigen Ansprüchen wiedergegeben.

Die Erfindung betrifft eine Schutzabdeckung für ein chirurgisches Instrument, insbesondere für ein Klammernahtgerät, umfassend eine einen Innenraum begrenzende Aussenumhüllung zur wenigstens teilweisen Abdeckung eines Kopfbereichs des chirurgischen Instruments, und eine Zugeinrichtung, welche mit der Aussenumhüllung kraftübertragend verbunden ist und ein Zugelement umfasst. Der Begriff "kraftübertragend" bezeichnet vorliegend eine Verbindung zwischen der Aussenumhüllung und der Zugeinrichtung, welche eine Übertragung der auf die Zugeinrichtung ausgeübten Kraft an die Aussenumhüllung ermöglicht.

Unter "Vorschubrichtung" wird diejenige Richtung verstanden, in der die Schutzabdeckung und das chirurgische Instrument in den Körper eines Patienten, bspw. in einen Darm vorgeschoben bzw. eingeschoben wird. Die Vorschubrichtung verläuft im Wesentlichen entlang einer Längsachse der Schutzabdeckung.

Ein distales Ende der Schutzabdeckung bezeichnet somit, in Vorschubrichtung gesehen, das vorderste Ende der Schutzabdeckung. Ein proximales Ende der Schutzabdeckung bezeichnet, in Vorschubrichtung gesehen, das hinterste Ende der Schutzabdeckung. Folglich bezeichnet der Begriff "distal" die vom Benutzer der erfindungsgemässen Vorrichtung abgewandte, ferner liegende Seite; dementsprechend bezeichnet der Begriff "proximal" die dem Benutzer, bspw. dem Chirurgen, zugewandte, näherliegende Seite. Der Vollständigkeit halber sei noch erwähnt, dass andere Definitionen der Begriffe "distal" und "proximal" im medizinalen Bereich gängig sind, welche allerdings keine Anwendung in der vorliegenden Anmeldung finden.

Das chirurgische Instrument, insbesondere das Klammernahtgerät oder der Zirkularstapler, weist üblicherweise eine proximale Betätigungseinrichtung, einen an die Betätigungseinrichtung angeschlossenen Schaftbereich und den an den Schaftbereich angeschlossenen Kopfbereich auf.

Das chirurgische Instrument kann ein zentral verlaufendes Lumen zur Aufnahme und Führung eines intraluminalen Instruments durch den Kopfbereich und ggf. den Schaftbereich aufweisen. Das intraluminale Instrument kann als eine Dornvorrichtung ausgebildet sein, welche aus dem Kopfbereich ausgefahren werden kann. Herkömmliche Dornvorrichtungen umfassen einen axial verlaufenden Dorn und einen an den Dorn angeschlossenen, axial verlaufenden Führungsbereich. Eine axial vom Kopfbereich abstehende, fest an dem Kopfbereich befestigte Dornvorrichtung ist ebenfalls möglich. In den Kopfbereich des chirurgischen Instruments, insbesondere des Klammernahtgeräts sind zirkulär angeordnete Klammern und ein zirkuläres Messer eingebaut. Das chirurgische Instrument weist eine Längsachse auf und ist wenigstens annähernd rotationsymmetrisch zur Längsachse ausgebildet, um das Einführen des chirurgischen Instruments in den Körper des Patienten zu ermöglichen.

Zudem weist das chirurgische Instrument ein Gegenstück auf, welches dazu bestimmt ist, bei einer Operation vor einer eigentlichen Verbindung von zwei Hohlorganteilen mittels Klammerung, insbesondere Darmhälften, in dem Hohlorganteil eingenäht zu werden, welcher von den zwei Hohlorganteilen dem chirurgischen Instrument gegenüberliegend angeordnet ist. Das Gegenstück ist weiter dazu bestimmt, den Dorn in einen Schaft des Gegenstücks aufzunehmen.

In einer bevorzugten Ausführungsform weist die Aussenumhüllung einen halbschalenförmigen, vorzugsweise im Wesentlichen halbkugelförmigen Abdeckbereich auf, welcher zentriert auf der Längsachse und nach proximal geöffnet ist. Der Abdeckbereich kann sich weiter nach proximal parallel zur Längsachse erstrecken und einen Mantelabschnitt umfassen, vorzugsweise in der Form einer zylindrischen, sich nach proximal erstreckenden Mantelwand. Die Bereitstellung des Mantelabschnitts verleiht dem Abdeckbereich die Form einer Hülse, welche eine stabile Aufnahme des chirurgischen Instruments in die Schutzabdeckung ermöglicht.

In einer bevorzugten Ausführungsform ist die Aussenumhüllung rotationssymmetrisch um die Längsachse ausgebildet, um eine anatomisch optimale Form zu haben, insbesondere im Hinblick auf eine Einführung in den Darm, und Reibungen bei der Einführung zu reduzieren.

Die Aussenumhüllung weist eine Trennlinie auf, welche die Aussenumhüllung in wenigstens ein erstes Segment und ein zweites Segment teilt. Ferner umfasst die Aussenumhüllung ein Verbindungsstück, das das erste Segment und das zweite Segment miteinander verbindet. Das erste Segment und das zweite Segment sind mit Hilfe einer Zugeinrichtung von einer Abdeck-Position in eine Freigabe-Position des Kopfbereichs überführbar, wenn eine Zugkraft in Richtung nach proximal auf die Zugeinrichtung ausgeübt wird. Das Verbindungsstück dient dazu, die vorzeitige Trennung der Aussenumhüllung in der Abdeck-Position zu vermeiden, bspw. bei der Einführung der auf dem chirurgischen Instrument aufgesetzten Schutzabdeckung in den Darm.

In einer bevorzugten Ausführungsform ist die Trennlinie als Schlitz ausgebildet, welcher die Aussenumhüllung in das wenigstens erste Segment und das zweite Segment teilt und deren Trennung bei der Überführung von der Abdeck-Position in die Freigabe-Position unterstützt.

Bevorzugt ist die Trennlinie durchstechend ausgebildet, d.h. dass die Trennlinie von einer Innenseite der Aussenumhüllung zu einer Aussenseite der Aussenumhüllung verläuft. Diese Ausführungsform ermöglich eine einfache Trennung der Aussenumhüllung. Allerdings ist es auch denkbar die Trennlinie als eine durchgehende Sollbruchstelle auszubilden, wobei mindestens eine Restdicke der Aussenumhüllung in der Form eines Films oder einer Kerbe die Sollbruchstelle darstellt.

In einer bevorzugten Ausführungsform verläuft die Trennlinie in einer die Längsachse enthaltende Mittelebene, um eine symmetrische Trennung der Aussenumhüllung in das erste Segment und das zweite Segment zu ermöglichen. Diese Ausführungsform unterstütz eine symmetrische Verteilung der auf die Aussenumhüllung ausgeübten Zugkraft, sodass ein Rutschen der Schutzabdeckung auf das chirurgische Instrument verhindert wird.

Das Verbindungsstück bildet eine Sollbruchstelle, welche dazu bestimmt ist, bei der Überführung von der Abdeck-Position in die Freigabe-Position zu brechen. Die Sollbruchstelle ist so ausgestaltet, dass sie ab einer bestimmten Zugkraft aufreisst.

In einer bevorzugten Ausführungsform kann das Verbindungsstück Stege umfassen. Die Stege verbinden das erste Segment und das zweite Segment quer zur Trennlinie, vorzugsweise in Umfangsrichtung der Aussenumhüllung, und sind, in radialer Richtung gesehen, vorzugsweise einander gegenüberliegend angeordnet. Diese Anordnung unterstützt weiter eine symmetrische Verteilung der Zugkraft bei der Überführung in die Freigabe-Position. Die Ausbildung des Verbindungsstücks ist derart gewählt, dass beim Ziehen der Zugeinrichtung nach proximal, d.h. in Richtung gegen die Vorschubrichtung, eine stossartige Bewegung beim Reissen der Sollbruchstelle minimiert wird.

In einer bevorzugten Ausführungsform umfasst das Verbindungsstück zwei Stege, d.h. ein Paar Stege, welche sich jeweils zwischen das erste Segment und das zweite Segment quer zur Trennlinie in Umfangsrichtung der Aussenumhüllung erstrecken und, in radialer Richtung gesehen, einander gegenüberliegend angeordnet sind. Diese Anordnung ermöglicht eine besonders einfache Herstellung sowie eine minimale Kraft zur Trennung der Aussenumhüllung.

In einer bevorzugten Ausführungsform kann das Verbindungsstück mehr als zwei Stege umfassen, bevorzugt zwei Paare Stege, besonders bevorzugt drei Paare Stege, welche sich paarweise jeweils zwischen das erste Segment und das zweite Segment quer zur Trennlinie in Umfangsrichtung der Aussenumhüllung erstrecken und, in radialer Richtung gesehen, einander gegenüberliegend angeordnet sind. Diese Anordnung ermöglicht eine schrittweise Trennung der Sollbruchstelle und minimiert somit eine stossartige Bewegung beim Reissen der Sollbruchstelle. Ferner kann die Anzahl der Stege je nach gewünschter Kraft zur Trennung der Aussenumhüllung gewählt werden.

Es ist ebenfalls denkbar, die Stege als perforierte Abschnitte auszubilden, welche sich paarweise jeweils zwischen das erste Segment und das zweite Segment quer zur Trennlinie erstrecken, wobei die Perforationen die Dicke des Stegs durchdringen oder sich nur über einen Teil der Dicke erstrecken. Diese Lösung hilft ebenfalls, stossartige Bewegungen beim Reissen weiter zu minimieren.

In der Abdeck-Position liegen durch die Trennlinie definierte Lippen des ersten Segments und des zweiten Segments wenigstens annähernd an und die Aussenumhüllung begrenzt somit den Innenraum zur wenigstens teilweisen Aufnahme des Kopfbereichs des chirurgischen Instruments.

Eine Ausbildung der Aussenumhüllung in drei, vier oder eine Vielfalt Segmente ist ebenfalls möglich. Dies kann von Vorteil sein, wenn kleinere Segmente für die Überführung von der Abdeck-Position in die Freigabe-Position durch die Form des chirurgischen Instruments erfordert werden, bspw. wegen dessen Asymmetrie. Die Ausbildung der Trennlinie und des Verbindungsstücks wird entsprechend angepasst, insbesondere die Anzahl der Stege und deren Anordnung zur Unterstützung der Überführung in die Freigabe-Position.

In einer bevorzugten Ausführungsform sind Ecken, welche am Schnittpunkt zwischen den Lippen des ersten Segments und des zweiten Segments und einem proximalen Rand der Aussenumhüllung ausgebildet abgerundet. Die Abrundung der Lippen dient dazu, Verletzungen beim Herausziehen der Schutzabdeckung zu vermeiden. Besonders bevorzugt verlaufen proximale Endabschnitte der Lippen nach proximal mit, im Umfangrichtung gemessen, zunehmendem Abstand von der Mittelebene und in der Form eines konkaven zur Mittelebene M ausgebildeten Viertelkreises. Diese Ausbildung minimiert weiter das Risiko einer Verletzung.

Erfindungsgemäss umfasst die Aussenumhüllung eine in ihrem distalen Scheitelbereich angeordnete, auf die Längsachse zentrierte Orientierungsvorrichtung, welche als eine Verdickung des Scheitelbereichs ausgebildet ist.

Die Verdickung des Scheitelbereichs verleiht dem Scheitelbereich eine höhere Steifigkeit als der Rest der Aussenumhüllung, und ermöglicht so eine Orientierungsfunktion. Die Orientierungsvorrichtung ist somit so ausgebildet, dass sie steifer als die restliche Aussenumhüllung ist. Folglich ist es möglich, taktil den Ort zu ertasten, an dem der Dorn beim vollständigen Ausfahren die zuvor verschlossene Organwand durchstossen wird. Ferner übt der verdickte Scheitelbereich einen mechanischen Widerstand aus, wenn der Kopfbereich des chirurgischen Instruments bei der Suche des Gegenstücks bewegt wird. In diesem Fall kann die geplante axiale Lage des Dorns angepasst werden, um optimal in das Gegenstück eingeführt zu werden. Dabei werden Verletzungen von Organen während der Suche nach der optimalen Position vermieden. Ferner wird das Risiko minimiert, dass der Dorn die Flanken der Orientierungsvorrichtung durchsticht.

Die Orientierungsvorrichtung kann somit dazu bestimmt sein, den Ort des Durchstechens des Dorns im Organ zu ertasten. Die Aussenumhüllung wird nämlich so auf den Kopfbereich aufgesetzt, dass die Orientierungsvorrichtung sich in der Verlängerung der Richtung des auszufahrenden Dorns befindet. Somit gibt die Orientierungsvorrichtung einen Hinweis auf den Ort des Durchstechens des Dorns, wenn der Dorn noch im Kopfbereich geschützt ist. Es folgt, dass eine erhöhte Sicherheit während der Positionierung des Kopfbereichs erreicht wird.

Ferner kann die Orientierungsvorrichtung dazu dienen, den Dorn aufzunehmen, welcher vom Kopfbereich des in die Schutzabdeckung eingeführten chirurgischen Instruments axial ausgefahren wird. Dies kann bei einer besonderen Bedienung des chirurgischen Instruments vorteilhaft sein, bei der der Dorn mindestens teilweise ausgefahren wird, bevor die Schutzabdeckung aufgerissen und zurückgezogen wird. Über die Orientierungsvorrichtung bleibt die Aussenumhüllung auf den Dorn zentriert und stabil auf dem Kopfbereich des chirurgischen Instruments. Diese Anordnung ermöglicht ein sicheres Positionieren des chirurgischen Instruments an der Einsatzstelle.

In einer bevorzugten Ausführungsform kann die Aussenumhüllung eine auf die Längsachse zentrierte vorzugsweise kreisförmige Öffnung aufweisen, welche in die Orientierungsvorrichtung mündet. Diese Öffnung ermöglicht eine symmetrische Verteilung der Kraft beim Aufreissen der Aussenumhüllung.

In einer bevorzugten Ausführungsform steht die Orientierungsvorrichtung auf der dem Innenraum abgewandten Seite der Aussenumhüllung ab. Dies erhöht den mechanischen Widerstand auf das abgetastete Organ, wenn der Kopfbereich des chirurgischen Instruments beim Abtasten bewegt wird.

In einer bevorzugten Ausführungsform ist die Orientierungsvorrichtung als eine sich nach distal entlang der Längsachse prismenförmig oder zylinderförmig erstreckende Verdickung des Scheitelbereichs ausgebildet. Diese Ausführungsformen stellen ein einfaches Design dar, das günstig hergestellt werden kann. Bei einer prismenförmigen Ausführungsform bildet die Orientierungsvorrichtung eine, im Querschnitt gesehen, polygonförmige Fläche, deren Kantenlänge zwischen 0,5 mm und 12 mm sein kann. Die Anzahl Ecken sowie deren Form, bspw. abgerundet oder nicht abgerundet, gleichmässig oder ungleichmässig verteilt, symmetrisch oder unsymmetrisch verteilt können vorteilhafterweise gewählt werden, um eine einfache Herstellung, insbesondere beim Spritzgiessen, zu erlauben. Bei einer zylinderförmigen Ausführungsform bildet die Orientierungsvorrichtung eine, im Querschnitt gesehen, kreisförmige Fläche, deren Durchmesser zwischen 3 mm und 12 mm sein kann. Diese Ausführungsformen sind für die meisten Klammernähgerät geeignet.

In einer bevorzugten Ausführungsform ist die Orientierungsvorrichtung als eine gewölbte Verdickung des Scheitelbereichs ausgebildet, um den Widerstand bei der Einführung der Schutzabdeckung in den Körper zu reduzieren. Diese Ausführungsform trägt insbesondere zur besseren Steuerung des Kopfbereichs des chirurgischen Instruments bei, bspw. zur besseren Steuerung eines Staplerkopfes durch den engen Analkanal eines Patienten.

Vorzugsweise ist die Orientierungsvorrichtung sich nach distal verjüngend ausgebildet, vorzugsweise kegelstumpfförmig ausgebildet, um den Widerstand bei der Einführung der Schutzabdeckung in den Körper zu reduzieren. Im Längsschnitt gesehen kann die Orientierungsvorrichtung somit beliebig konisch ausgebildet sein.

Die Verdickung kann zumindest teilweise die Öffnung umgeben und bildet einen sich entlang der Längsachse der Schutzabdeckung erstreckenden Führungskanal. Der Führungskanal ist dazu bestimmt, die Steifigkeit der Orientierungsvorrichtung räumlich zu gestalten, um das Ertasten zu optimieren. Der Führungskanal kann gegebenenfalls auch den Dorn aufnehmen und führen, sodass die Schutzabdeckung in Richtung der Längsachse zentriert auf den Dorn bleibt.

Der Führungskanal ist von einer inneren Flanke der Verdickung umschlossen. Ferner weist die Verdickung eine an die innere Flanke anschliessende, auf der dem Führungskanal abgewandten Seite abfallend verlaufende äussere Flanke auf. Der Begriff "Flanke" definiert in diesem Kontext eine seitliche, gegebenenfalls geneigte Verdickungswand.

Der Führungskanal umfasst eine proximale Führungskanalöffnung auf der dem Innenraum zugewandten Ende des Führungskanals und eine distale Führungskanalöffnung auf der dem Innenraum abgewandten Ende des Führungskanals.

Besonders bevorzugt ist die innere Flanke direkt angrenzend an den Rand der Öffnung ausgebildet. Mit anderen Worten beginnt die innere Flanke direkt am Rand der Öffnung, sodass die proximale Führungskanalöffnung der Öffnung entspricht. Somit können keine Schmutzpartikeln und kein Gewebe am Rand der Öffnung ansammeln.

In einer bevorzugten Ausführungsform weist der Führungskanal eine Verengungsstelle auf, deren lichte Öffnung, in einer rechtwinklig zur Längsachse verlaufenden Ebene gesehen, so bemessen ist, dass sie einen Widerstandspunkt bei der Einführung des Dorns bilden kann. Konkret kann die lichte Öffnung kleiner als der grösste Querschnitt eines sich zuspitzenden Abschnitts des Dorns sein. Folglich kann sichergestellt werden, dass bei der Erreichung der Verengungsstelle während der Einführung des Dorns in den Führungskanal einen erhöhten Widerstand erzeugt wird, welcher als Signal bzw. Information über die axiale Position des Dorns ausgelegt werden kann. Dies ermöglicht ein sicheres Positionieren des chirurgischen Instruments, insbesondere des Dorns, an die Einsatzstelle.

In einer bevorzugten Ausführungsform ist die Verdickung in Form eines gewölbten Kegelstumpfs ausgebildet. Diese Form unterstützt eine einfache Einführung in den Körper.

In einer bevorzugten Ausführungsform ist ein Übergangsbereich der Verdickung, welcher sich zwischen der inneren Flanke und der äusseren Flanke erstreckt, abgeflacht oder abgerundet. Diese Ausbildung der Verdickung reduziert ebenfalls das Risiko einer Verletzung durch eckige Bereiche der Schutzabdeckung.

In einer bevorzugten Ausführungsform ist der Querschnitt des Führungskanals sich verjüngend ausgebildet, insbesondere sich kontinuierlich verjüngend, um die Steifigkeit der Orientierungsvorrichtung axial zu gestalten.

Besonders bevorzugt ist der Querschnitt des Führungskanals, in Vorschubrichtung gesehen, sich von der proximalen Führungskanalöffnung, in Vorschubrichtung gesehen, verjüngend ausgebildet. Die trichterförmige Ausbildung des Führungskanals ermöglicht eine weitere Form der Gestaltung der Steifigkeit der Orientierungsvorrichtung. Zudem unterstützt die trichterförmige Ausbildung des Führungskanals die Einführung des Dorns in den Führungskanal, wenn die Längsachse der Schutzabdeckung, d.h. des Führungskanals, nicht mehr ganz mit der axialen Richtung des Dorns übereinstimmt. Dies kann vorkommen bspw. bei der Einführung des chirurgischen Instruments in den Körper, wenn die auf dem chirurgischen Instrument in der Abdeck-Position aufgesetzte Schutzabdeckung sich seitlich bewegt hat. Während der Einführung des Dorns in den Führungskanal nimmt die Aussenumhüllung wieder ein auf die Längsachse des chirurgischen Instruments gerichtete Position ein, sodass die Schutzabdeckung optimal den Kopfbereich in der Abdeck-Position abdeckt.

Vorzugsweise ist der Querschnitt des Führungskanals, in Vorschubrichtung gesehen, sich konisch verjüngend ausgebildet. Diese Ausführungsform weist den weiteren Vorteil auf, dass ihre Herstellung, insbesondere in einem Spritzgussverfahren, einfach bewerkstelligt werden kann.

In einer bevorzugten Ausführungsform verjüngt sich der Querschnitt des Führungskanals in der Form eines Konus auf, um eine symmetrische Ausbildung des Führungskanals und gegebenenfalls eine genaue Führung zu ermöglichen.

In einer bevorzugten Ausführungsform teilt die Trennlinie die Orientierungsvorrichtung in ein erstes Orientierungselement und ein zweites Orientierungselement, wobei das erste Orientierungselement mit dem ersten Segment und das zweite Orientierungselement mit dem zweiten Segment verbunden sind. Beim Herausziehen der Schutzabdeckung wird somit sichergestellt, dass die sich in zwei Teile trennende Orientierungsvorrichtung kein verhakendes Hindernis darstellt und zusammen mit den jeweiligen Orientierungselementen entfernt werden kann.

In einer bevorzugten Ausführungsform erstreckt sich das Verbindungsstück ebenfalls zwischen dem ersten Orientierungselement und dem zweiten Orientierungselement. Diese Anordnung ermöglicht eine regelmässige Verteilung der Kraft zur Trennung der Aussenumhüllung. Ferner bleibt der Führungskanal erhalten, solange die Schutzabdeckung in der Abdeck-Position ist.

In einer bevorzugten Ausführungsform kann das Verbindungsstück ein Paar Stege zwischen dem ersten Orientierungselement und dem zweiten Orientierungselement und ein Paar Stege zwischen dem ersten Segment und dem zweiten Segment aufweisen. Diese Ausführungsform ermöglicht zusätzlich eine einfache Herstellung der Aussenumhüllung.

In einer bevorzugten Ausführungsform ist das Verbindungsstück nur zwischen dem ersten Orientierungselement und dem zweiten Orientierungselement ausgebildet. Somit sind nur das erste Orientierungselement und das zweite Orientierungselement durch das Verbindungsstück verbunden. Diese Anordnung stellt sicher, dass der Führungskanal erhalten bleibt, solange die Schutzabdeckung in der Abdeck-Position ist.

In einer bevorzugten Ausführungsform kann das Verbindungsstück nur ein Paar Stege zwischen dem ersten Orientierungselement und dem zweiten Orientierungselement aufweisen. Diese Ausführungsform ermöglicht zusätzlich eine besonders einfache Herstellung der Aussenumhüllung sowie eine optimale Kraft zur Trennung der Aussenumhüllung. In einer bevorzugten Ausführungsform umfasst die Zugeinrichtung ein weiteres Zugelement. Vorzugsweise verlaufen das Zugelement und das weitere Zugelement in eine longitudinale Ebene, welche die Längsachse der Schutzabdeckung enthält. Dies ermöglicht eine symmetrische Verteilung der Zugkraft auf die Aussenumhüllung. Somit wird vermieden, dass die Schutzabdeckung beim Ziehen vom Kopfbereich des chirurgischen Instruments rutscht.

Vorzugsweise verlaufen das Zugelement und das weitere Zugelement in einem Abstand zueinander, der einem Aussendurchmesser des Schaftbereichs des chirurgischen Instruments wenigstens annähernd entspricht. Somit bildet die Schutzabdeckung eine kompakte Einheit zusammen mit dem chirurgischen Instrument. Da die Zugelemente entlang des chirurgischen Instruments verlaufen, kann zudem die Zugkraft beim Herausziehen der Schutzabdeckung in Richtung der Längsachse des chirurgischen Instruments ausgeübt werden.

In einer bevorzugten Ausführungsform ist das Zugelement über einen Vorsprungsabschnitt mit dem ersten Segment und das weitere Zugelement über einen weiteren Vorsprungsabschnitt mit dem zweiten Segment verbunden. Vorzugsweise verlaufen der Vorsprungsabschnitt und der weitere Vorsprungsabschnitt in die longitudinale Ebene, um ebenfalls eine optimale Ausrichtung der Zugkraft zu unterstützen.

Der Vorsprungsabschnitt und der weitere Vorsprungsabschnitt können in ihrem proximalen Endbereich in einem Abstand zueinander verlaufen, der dem Aussendurchmesser des chirurgischen Instruments wenigstens annähernd entspricht, und in ihrem distalen Endbereich in einem Abstand zueinander verlaufen, der dem lichten Durchmesser der Aussenumhüllung wenigstens annähernd entspricht. Der Vorsprungsabschnitt und der weiteren Vorsprungsabschnitt bilden somit einen Übergangsbereich zur optimalen Verbindung der Zugelemente mit der Aussenumhüllung und Übertragung der Zugkraft. Dies erfolgt durch die Anpassung des Abstands zwischen den Zugelementen an den Abstand, welcher dem lichten Durchmesser der Aussenumhüllung entspricht.

In einer bevorzugten Ausführungsform sind das Zugelement und das weitere Zugelement über zwei beabstandet voneinander angeordnete, offene Ringe verbunden, welche jeweils in eine wenigstens annähernd rechtwinklig zur Längsachse der Schutzabdeckung verlaufende Ebene ausgebildet sind. Die Ringe sind dazu bestimmt, den Abstand zwischen dem Zugelement und dem weiteren Zugelement wenigstens annähernd konstant zu halten. Der Durchmesser der Ringe ist derart bemessen, dass er dem Aussendurchmesser des chirurgischen Instruments wenigstens annähernd entspricht. Das Zugelement und das weitere Zugelement bilden somit einen Aufnahmebereich für das chirurgische Instrument zwischen den Ringen, welcher das axiale Positionieren des chirurgischen Instruments in die Schutzabdeckung unterstützt. Die Ringe haben den zusätzlichen Vorteil, dass sie eine Verdrehung des Zugelements und des weiteren Zugelements, insbesondere in dem Aufnahmebereich, bei der Bedienung der Zugeinrichtung oder bei der Einführung in den Körper verhindern.

Vorzugsweise weisen die offenen Ringe jeweils eine Öffnung auf, die auf der gleichen Seite der durch das Zugelement und das weitere Zugelement definierten longitudinalen Ebene ausgebildet sein kann. Dies ermöglicht eine einfache Einführung des chirurgischen Instruments in den Aufnahmebereich.

In einer bevorzugten Ausführungsform verlaufen das Zugelement und das weitere Zugelement distal vom Aufnahmebereich in die longitudinale Ebene und proximal zum Aufnahmebereich in, nach proximal gesehen, zunehmendem Abstand von der longitudinalen Ebene. Mit anderen Worten, bei gebogenen Ausführungen des chirurgischen Instruments, insbesondere des Klammernahtgeräts, folgen das Zugelement und das weitere Zugelement einer Sehne des chirurgischen Instruments. Somit folgen die Zugelemente dem allgemeinen Längsprofil des chirurgischen Instruments und es wird verhindert, dass die Zugelemente zu weit von der Betätigungseinrichtung des chirurgischen Instruments entfernt liegen.

In einer bevorzugten Ausführungsform sind das Zugelement und das weitere Zugelement an ihren proximalen Enden über einen vorzugsweise halbkreisförmigen Bindungsabschnitt zusammen verbunden. Der Bindungsabschnitt trägt weiter dazu bei, ein Verhaken der Zugeinrichtung in dem chirurgischen Instrument zu verhindern. Ferner bildet der Bindungsabschnitt einen optimalen Bereich, um die Zugkraft wenigstens annähernd axial auszuüben, und somit ein Verkippen der Schutzabdeckung vom Kopfbereich zu vermeiden.

In einer bevorzugten Ausführungsform verlaufen die Mittelebene und die longitudinale Ebene rechtwinklig zueinander. Somit kann eine zur Mittelebene symmetrische Verteilung der auf die Zugeinrichtung ausgeübten Zugkraft auf die Sollbruchstellen erreicht werden, während die Aussenumhüllung möglichst zentriert auf die Längsachse bleibt.

In einer bevorzugten Ausführungsform ist die Aussenumhüllung rotationssymmetrisch um die Längsachse ausgebildet. Dies ermöglicht eine anatomisch optimale Einführung der Schutzabdeckung in den Körper sowie eine einfache Herstellung der Schutzabdeckung.

In einer bevorzugten Ausführungsform ist die Schutzabdeckung einstückig geformt. Der Begriff "einstückig" ist so zu verstehen, dass die Schutzabdeckung einen einzigen Teil bildet, welcher bspw. durch Spritzgiessen hergestellt werden kann, und keine Naht vorliegt. Dies ist besonders von Vorteil, um Schnittstellen zu vermeiden, in welche keine Schmutzpartikel und kein Gewebe ansammeln können. Somit kann gleichzeitig eine sichere und kostengünstige Vorrichtung hergestellt werden.

Zudem kann die Schutzabdeckung integral ausgebildet sein, d.h. aus dem gleichen Material gefertigt sein. Dies unterstützt weiter die einfache und kostengünstige Herstellung der Schutzabdeckung.

Die Schutzabdeckung kann aus einem elastisch und/oder plastisch verformbaren Material wie einem thermoplastischen

Elastomer (TPE), einem Kautschuk oder einem Thermoplast geformt sein, welches für medizinische Anwendungen geeignet ist. Solche Materialien sind dem Fachmann bekannt.

In einer bevorzugten Ausführungsform weist die Oberfläche der Aussenumhüllung zungenförmige, sich vom distalen zum proximalen Ende der Aussenumhüllung erstreckende, in Umfangsrichtung der Aussenumhüllung vorzugsweise gleichmässig verteilte und vorzugsweise gleich ausgebildete Wölbungen auf. Die Wölbungen bewirken eine Ausrichtung der Aussenumhüllung im Wesentlichen parallel zur Einschubrichtung zur Erleichterung der Einführung der Schutzabdeckung in den Körper auf. Ferner reduzieren die Wölbungen das Risiko, dass sich der Kopfbereich bei der Einführung des chirurgischen Instruments in Gewebefalten verfängt. Folglich sitzt die Aussenumhüllung stabiler auf dem Kopfbereich des chirurgischen Instruments bei dessen Einführung. Die gewölbte Struktur der Aussenumhüllung reduziert den Gewebswiderstand beim Einführen des Kopfbereichs des chirurgischen Instruments, insbesondere eines Staplerkopfes, und trägt somit zur besseren Steuerung des Kopfbereichs des chirurgischen Instruments bei, bspw. zur besseren Steuerung des Staplerkopfes durch den engen Analkanal eines Patienten.

In einer bevorzugten Ausführungsform umfasst die Aussenumhüllung eine im Innenraum angeordnete, umlaufende Stützkontur zur axialen Positionierung des Kopfbereichs. Die Stützkontur ist dazu bestimmt, den axialen Abstand zwischen dem Kopfbereich und der Öffnung über die axiale Positionierung des Kopfbereichs in der Aussenumhüllung zu bestimmen. Somit kann die Position des distalen Endes des Dorns, bspw. im ausgefahrenen Zustand, bezüglich der Öffnung und ggf. der distalen Führungskanalöffnung präzis vorbestimmt und bekannt sein. Beim Erreichen der Stützkontur wird zudem sichergestellt, dass der Kopfbereich in die Aussenumhüllung ausreichend eingeführt ist, sodass die Aussenumhüllung nicht verkippt.

Die Stützkontur ist in Kontakt mit dem Kopfbereich über eine Stützkonturfläche, welche so bemessen ist, dass beim Ziehen der Schutzabdeckung nach proximal die Aussenumhüllung und somit die Schutzabdeckung wenigstens annähernd ohne stossartige Bewegung beim Rutschen über den Kopfbereich erfolgt.

In einer bevorzugten Ausführungsform ist die Stützkontur in der Form von radial nach innen abstehenden, im Umfangsrichtung der Aussenumhüllung gleichmässig verteilten und vorzugsweise gleich ausgebildeten Vorsprüngen, deren proximale Endflächen in einer rechtwinklig zur Längsachse verlaufende Stützkonturebene liegen und die Stützkonturfläche bilden. Wie bereits erwähnt bestimmt die Stützkontur die axiale Position des Kopfbereichs in der Aussenumhüllung. Die als eine Vielfalt Vorsprünge ausgebildete Stützkontur ermöglicht ein Ziehen der Abdeckung nach proximal wenigstens annähernd ohne stossartige Bewegung beim Rutschen über den Kopfbereich.

Der lichte Durchmesser der Aussenumhüllung proximal zur Stützkonturebene entspricht wenigstens annähernd dem Durchmesser des Kopfbereichs, um eine zentrierte und stabile Positionierung des chirurgischen Instruments, insbesondere des Kopfbereichs sicherzustellen.

In einer bevorzugten Ausführungsform ist die Stützkontur als einen in Umfangsrichtung verlaufenden Wulst ausgebildet. Die Stützkontur ist in Kontakt mit dem Kopfbereich über die Stützkonturfläche, welche so bemessen ist, dass der lichte Durchmesser der Aussenumhüllung proximal zur Stützkonturebene dem Durchmesser des Kopfbereichs eines ersten chirurgischen Instruments und der lichte Durchmesser der Aussenumhüllung distal zur Stützkonturebene dem Durchmesser des Kopfbereichs eines zweiten chirurgischen Instruments entspricht, wobei der Durchmesser des Kopfbereichs des zweiten chirurgischen Instruments kleiner als der Durchmesser des Kopfbereichs des ersten chirurgischen Instruments ist. Somit passt eine Grösse der Aussenumhüllung auf wenigstens zwei Grössen des Kopfes eines chirurgischen Instruments.

Die Stützkontur, insbesondere der Wulst, kann durch diskrete und gleichmässig oder ungleichmässig am Umfang verteilte Vorsprünge oder auch durch geschlossene Konturen gebildet werden. Diese Ausbildungen ermöglichen ebenfalls die Aufnahme von zwei unterschiedlichen Durchmessern der Kopfbereiche.

Vorzugsweise ist die Oberfläche des Innenraums distal zur Stützkonturebene glatt, d.h. unter anderen frei von Vorsprüngen oder Rippen, um eine einfache Herstellung und eine gleichmässige Verteilung der Kräfte zu ermöglichen.

In einer bevorzugten Ausführungsform ist das Verbindungsstück distal zur Stützkonturebene angeordnet. Somit erfolgt das Reissen des Verbindungsstücks, d.h. der Sollbruchstelle erst beim Rutschen der Stützkontur auf den Kopfbereich, wenn die Schutzabdeckung nach proximal gezogen wird.

Als erster Schritt bei der Verwendung der Schutzabdeckung wird das chirurgische Instrument, insbesondere das Klammernahtgerät oder der Zirkularstapler, in die Schutzabdeckung eingeführt, bis der Kopfbereich die Stützkontur erreicht hat. Mit anderen Worten wird der Kopfbereich in die Aussenumhüllung und der Schaftbereich in den Aufnahmebereich eingeführt, sodass die Zugeinrichtung nach proximal entlang der Längsachse des chirurgischen Instruments verläuft. Dabei verlaufen die Längsachse des chirurgischen Instruments und der Schutzabdeckung wenigstens annähernd aufeinander. Die Schutzabdeckung ist in der Abdeck-Position auf dem Kopfbereich zentriert.

In einem zweiten Schritt werden der Kopfbereich und ggf. teilweise der Schaftbereich in den Körper, bspw. in den Darm, eingeführt. Ein Operateur führt den Kopfbereich einschliesslich das intraluminale Instrument in den Darm ein und schiebt das chirurgische Instrument in Vorschubrichtung zur Einsatzstelle vorwärts.

Aufgrund der abgerundeten Form der Schutzabdeckung, die gleichzeitig als Abdeckung des Kopfbereichs dient, werden keine Gewebefalten, insbesondere Schleimhautfalten mitgeschoben, gedehnt und somit verletzt. Ausserdem ist die Schutzabdeckung auf dem Kopfbereich über die Mantelwand gehalten, wodurch Verletzungen durch den Kopfbereich, bspw. durch Ränder des Kopfbereichs auch vermieden werden.

Beim Einführen und Vorschieben des chirurgischen Instruments muss vom Operateur darauf geachtet werden, dass die Zugeinrichtung aus dem Darm zur jeden Zeit heraushängt. Dies wird durch die zwischen den Zugelementen angeordneten Ringe und den Bindungsabschnitt am Ende der Zugelemente optimal unterstützt.

Das chirurgische Instrument wird bis zu einem gewünschten Abstand zum Operationsort vorgeschoben. Dabei kann die Orientierungsvorrichtung der Schutzabdeckung verwendet werden, um den Ort des Durchstechens des intraluminalen Instruments, vorliegend des Dorns, im Darm zu ertasten.

In einem weiteren Schritt wird die Schutzabdeckung aufgerissen und zurückgezogen. Dabei zieht der Operateur die Zugeinrichtung über die Zugelemente nach proximal. Der Druck, der ausgeübt wird, ist ausreichend gross zu wählen, um die Sollbruchstellen, d.h. das Verbindungsstück, aufzureissen. Dabei werden das das erste Segment und das erste Orientierungselement von dem zweiten Segment und dem zweiten Orientierungselement getrennt. Somit wird die Aussenumhüllung in zwei Hälfte auseinandergetrennt und in die Freigabe-Position überführt.

Der Operateur zieht nach Positionierung des chirurgischen Instruments vorzugsweise die Schutzabdeckung so weit zurück, bis der Kopfbereich frei ist. Da der Darm in radialer Richtung leicht dehnbar ist, rutschen die beiden Hälfte jeweils mittels der Zugkraft zwischen dem Kopfbereich und der Innenwand des Darms hindurch und gleiten nach proximal. Um noch bei dieser Bewegungsrichtung eine Verletzung des Darms bzw. der Darmschleimhaut zu vermeiden, sind sämtliche Kanten, bspw. die Lippen des ersten Segments und des zweiten Segments in ihrem proximalen Endabschnitt, abgerundet ausgebildet.

Im nächsten Schritt fährt der Operateur das intraluminale Instrument aus dem Inneren des Kopfbereichs heraus. Der Dorn perforiert eine anliegende Darmwand an gewünschter Stelle. Auf den ausgefahrenen Dorn wird nun der Schaft des Gegenstücks (eine pilzförmig gestaltete Gegendruckplatte) aufgesteckt.

Weiter wird der Dorn zurückgefahren und die Gefässhälften zusammengespannt, sodass eine zirkuläre Naht zur Durchführung einer Anastomose geklammert werden kann. Wenn die Naht erzeugt und die anteiligen Gefässanteile in Ringform mit dem zirkulären Messer ausgeschnitten sind, wird das chirurgische Instrument zusammen mit der Schutzabdeckung zurückgezogen.

Es wird somit ein Verfahren zur Durchführung einer Anastomose, in welcher zwei Gefässhälften eingenäht werden, mit einem chirurgischen Instrument an einem Operationsort, insbesondere mit einem Klammernahtgerät oder einem Zirkularstapler offenbart, umfassend die Schritte:
a) Bereitstellung des chirurgischen Instruments und einer Schutzabdeckung für das chirurgische Instrument, insbesondere für das Klammernahtgerät, gemäss einer der oben offenbarten Ausführungsform;
b) Einführung eines Kopfbereichs des chirurgischen Instruments in eine Aussenumhüllung der Schutzabdeckung und eines Schaftbereichs des chirurgischen Instruments in einen Aufnahmebereich der Schutzabdeckung, wobei eine Zugeinrichtung der Schutzabdeckung nach proximal entlang der Längsachse des chirurgischen Instruments verläuft und die Schutzabdeckung in einer Abdeck-Position auf dem Kopfbereich zentriert ist;
c) Einführung des die Schutzabdeckung tragenden Kopfbereichs und ggf. teilweise des Schaftbereichs in den Körper eines Patienten, bspw. in eine erste Gefässhälfte des Darms, einschliesslich eines intraluminalen Instruments des chirurgischen Instruments, bspw. einer Dornvorrichtung, und Einschieben bis zu einem gewünschten Abstand zum Operationsort, wobei das chirurgische Instrument ein zentral verlaufendes Lumen zur Aufnahme und Führung des intraluminalen Instruments durch den Kopfbereich und ggf. den Schaftbereich aufweisen kann;
d) Ertasten des Orts des Durchstechens des intraluminalen Instruments im Darm über die Orientierungsvorrichtung der Schutzabdeckung zur Positionierung des Kopfbereichs und Positionierung des Kopfbereichs am Operationsort;
e) Aufreissen der Schutzabdeckung und Überführung der Schutzabdeckung in eine Freigabe-Position des Kopfbereichs;
f) Rückzug der Schutzabdeckung nach proximal über die Zugeinrichtung bis der Kopfbereich frei ist;
f) Herausfahren des intraluminalen Instruments aus dem Inneren des Kopfbereichs;
g) Perforierung durch das intraluminale Instrument einer anliegenden Darmwand der ersten Gefässhälfte an gewünschter Stelle und Aufsetzen eines Gegenstücks auf das intraluminale Instrument, welches Gegenstück vor einer eigentlichen Verbindung einer zweiten Gefässhälfte mit der ersten Gefässhälfte mittels Klammerung in die zweite Gefässhälfte des Darms eingenäht wurde;
h) Zurückfahren des intraluminalen Instruments und Zusammenspannung der beiden Gefässhälften, um eine zirkuläre Naht mithilfe von Klammern zu bilden und die Anastomose abzuschliessen.

Die Verdickung des Scheitelbereichs der Orientierungsvorrichtung verleiht dem Scheitelbereich eine höhere Steifigkeit als der Rest der Aussenumhüllung, und ermöglicht so eine Orientierungsfunktion. Folglich ist es möglich, taktil über die Orientierungsvorrichtung den Ort zu ertasten, an dem der Dorn beim vollständigen Ausfahren die zuvor verschlossene Organwand durchstossen wird. Ferner übt der verdickte Scheitelbereich einen mechanischen Widerstand aus, wenn der Kopfbereich des chirurgischen Instruments bei der Suche des Gegenstücks bewegt wird. In diesem Fall kann die geplante axiale Lage des Dorns angepasst werden, um optimal in das Gegenstück eingeführt zu werden. Dabei werden Verletzungen von Organen während der Suche nach der optimalen Position vermieden. Ferner wird das Risiko minimiert, dass der Dorn die Flanken der Orientierungsvorrichtung durchsticht.

Der Vollständigkeit halber sei noch erwähnt, dass Abweichungen zu den oben beschriebenen Schritten, bspw. eine andere Reihenfolge, je nach Umständen auch vorstellbar sind.

Weitere Vorteile und Eigenschaften der Erfindung gehen aus der nachstehenden Beschreibung ausgewählter Ausführungsbeispiele hervor, welches anhand der beiliegenden Figuren erläutert wird.

### Beschreibung der Figuren

Die Figuren zeigen rein schematisch:
- Fig. 1: eine perspektivische Ansicht einer erfindungsgemässen Schutzabdeckung für ein chirurgisches Instrument;
- Fig. 2: eine Draufsicht der Schutzabdeckung gemäss Fig. 1;
- Fig. 3: eine Seitenansicht der Schutzabdeckung gemäss Fig. 1;
- Fig. 4: eine Frontalansicht der Schutzabdeckung gemäss Fig. 1;
- Fig. 5: einen Querschnitt entlang der Linie V-V durch eine Aussenumhüllung der Schutzabdeckung gemäss Fig. 1;
- Fig. 6: einen Querschnitt entlang der Linie VI-VI durch die Aussenumhüllung der Schutzabdeckung gemäss Fig. 1;
- Fig. 7: einen Querschnitt entlang der Linie V-V durch die Schutzabdeckung gemäss Fig. 1, in welcher ein chirurgisches Instrument eingeführt ist;
- Fig. 8: den Querschnitt der Fig. 7, in welchem ein Dorn des chirurgischen Instruments bis zu einer Orientierungsvorrichtung der Schutzabdeckung ausgefahren ist;
- Fig. 9: den Querschnitt der Fig. 7, in welchem die Schutzabdeckung entlang des chirurgischen Instruments teilweise gezogen wurde;
- Fig. 10: eine Seitenansicht der Schutzabdeckung und des in der Schutzabdeckung eingeführten chirurgischen Instruments; und
- Fig. 11: einen dem Querschnitt der Fig. 6 ähnlichen Querschnitt durch die Aussenumhüllung der Schutzabdeckung einer weiteren Ausführungsform;
- Fig. 12: einen dem Querschnitt der Fig. 5 ähnlichen Querschnitt durch die Aussenumhüllung der Schutzabdeckung gemäss Fig. 11 in leicht vergrösserter Ansicht.

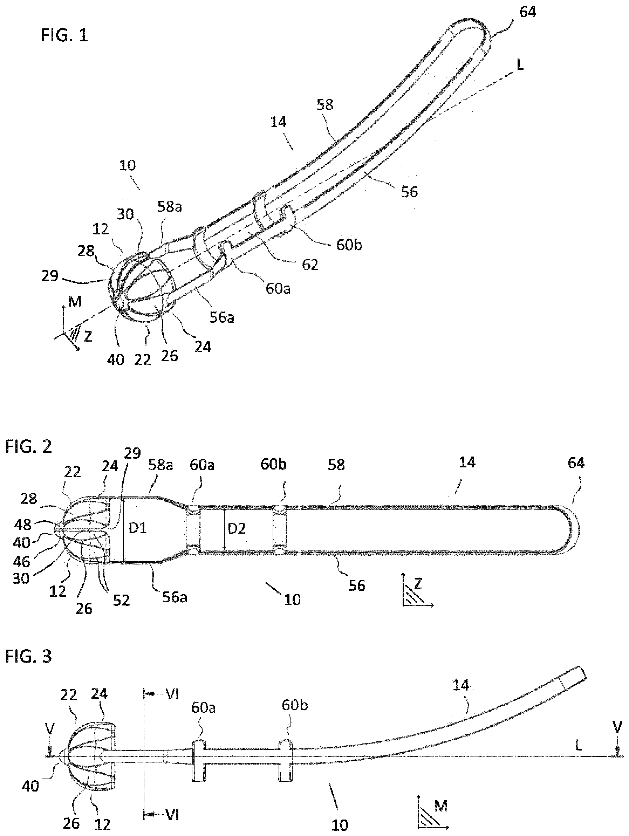

Die in Fig. 1 bis Fig. 6 dargestellte Schutzabdeckung 10 für ein chirurgisches Instrument 11, insbesondere für ein Klammernahtgerät, umfasst eine Aussenumhüllung 12 zur wenigstens teilweisen Abdeckung eines Kopfbereichs 13 des chirurgischen Instruments, und eine Zugeinrichtung 14, welche mit der Aussenumhüllung 12 kraftübertragend verbunden ist.

Die Aussenumhüllung 12 weist einen halbschalenförmigen, vorliegend im Wesentlichen halbkugelförmigen Abdeckbereich 22 auf, welcher zentriert auf einer Längsachse L der Schutzabdeckung und nach proximal geöffnet ist. Die Aussenumhüllung 12 umfasst ferner einen Mantelabschnitt, welcher an einen proximalen Rand des Abdeckbereichs 22 angeschlossen ist und vorliegend in der Form einer zylindrischen, sich nach proximal erstreckenden Mantelwand 24 verläuft. Die Aussenumhüllung 12 ist somit rotationssymmetrisch um die Längsachse L ausgebildet.

Vorliegend ist die Aussenumhüllung 12 aus einem ersten Segment 26 und einem zweiten Segment 28 gebildet, welche jeweils auf einer Seite einer die Längsachse L enthaltenden Mittelebene M liegen und durch eine in die Mittelebene M verlaufenden Trennlinie 29 getrennt sind. Vorliegend ist die Trennlinie als ein Schlitz 29 ausgebildet. Somit stellen das erste Segment 26 und das zweite Segment 28 je eine Hälfte der Aussenumhüllung 12 dar.

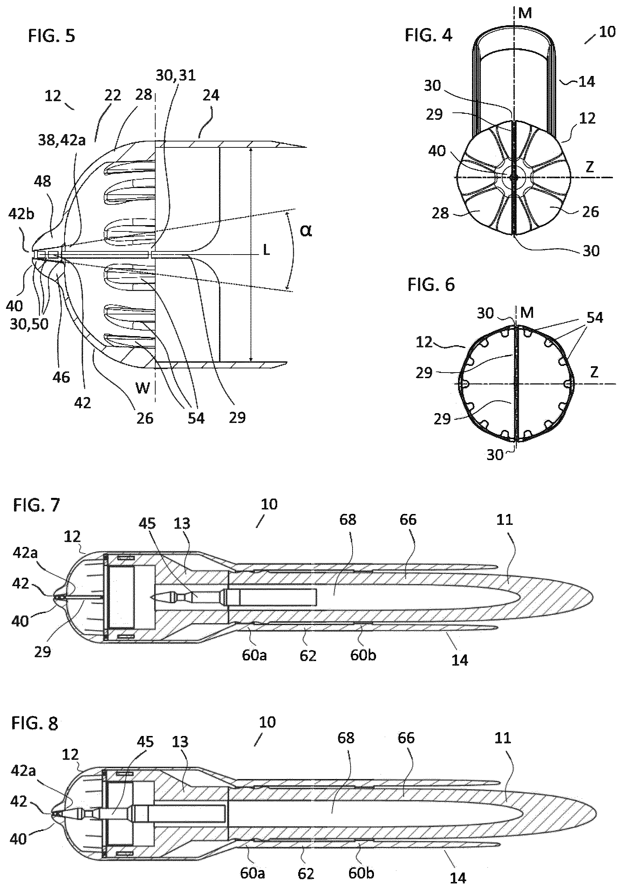

Das erste Segment 26 und das zweite Segment 28 sind durch ein Verbindungsstück 30 in der Form von zwei Stege 31 miteinander verbunden. Die Stege 31 verbinden das erste Segment 26 und das zweite Segment 28 in Umfangsrichtung und sind, in radialer Richtung gesehen, einander gegenüberliegend im Abdeckbereich 22 angeordnet, wie dies auch in Fig. 5 ersichtlich ist.

In einer Abdeck-Position begrenzt somit die Aussenumhüllung 12 einen Innenraum zur wenigstens teilweisen Aufnahme des Kopfbereichs des chirurgischen Instruments.

Proximal zum Verbindungsstück 30 weitet sich der Schlitz 29 in Umfangsrichtung kontinuierlich auf und verläuft auf beide Seiten der Mittelebene M in der Form eines symmetrischen und konkaven zur Mittelebene M ausgebildeten Viertelkreises. Somit werden eckige Kanten im proximalen Endbereich der Aussenumhüllung 12 vermieden.

In ihrem distalen Scheitelbereich weist die Aussenumhüllung 12 eine auf die Längsachse L zentrierte Öffnung 38 auf.

Ferner umfasst die Aussenumhüllung 12 eine um die Öffnung 38 angeordnete Orientierungsvorrichtung 40, welche als eine Verdickung 40 des Scheitelbereichs ausgebildet ist. Die Orientierungsvorrichtung 40 ist als eine wulstförmige, in Vorschubrichtung gesehen, sich verjüngende Verdickung 40 ausgebildet, welche auf der dem Innenraum abgewandten Seite der Aussenumhüllung 12 absteht.

Die Verdickung 40 umgibt die Öffnung 38 und bildet einen sich entlang der Längsachse L erstreckenden Führungskanal 42.

Der Führungskanal 42 ist von einer inneren Flanke der Verdickung 40 umschlossen. Ferner weist die Verdickung 40 eine an die innere Flanke anschliessende, auf der dem Führungskanal 42 abgewandten Seite abfallend verlaufende äussere Flanke auf.

Der Führungskanal umfasst eine proximale Führungskanalöffnung 42a auf der dem Innenraum zugewandten Ende des Führungskanals und eine distale Führungskanalöffnung 42b auf der dem Innenraum abgewandten Ende des Führungskanals 42, wobei die innere Flanke direkt angrenzend an den Rand der Öffnung 38 ausgebildet ist. Der Querschnitt des Führungskanals 42 ist, in Vorschubrichtung gesehen, sich kontinuierlich verjüngend in der Form eines Konus ausgebildet. In der abgebildete Ausführungsform bildet die Mantellinie des Konus einen Winkel α (alpha) von 16 Grad. Die Höhe der Verdickung beträgt, von der Öffnung 38 gemessen, ca. 5 mm.

Die Orientierungsvorrichtung 40 ist aus einem ersten Orientierungselement 46 und einem zweiten Orientierungselement 48 gebildet, welche ebenfalls jeweils auf einer Seite der Mittelebene M liegen und durch den in die Mittelebene M verlaufenden Schlitz 29 getrennt sind. Somit stellen das erste Orientierungselement 46 und das zweite Orientierungselement 48 je eine Hälfte der Orientierungsvorrichtung 40 dar. Das erste Orientierungselement 46 ist mit dem ersten Segment 26 und das zweite Orientierungselement 48 ist mit dem zweiten Segment 28 einstückig ausgebildet.

Das erste Orientierungselement 46 und das zweite Orientierungselement 48 sind ebenfalls durch das Verbindungsstück in der Form von drei Paar weiteren Stegen 50 miteinander verbunden. Die Stege 50 verbinden das erste Orientierungselement 46 und das zweite Orientierungselement 48 in Umfangsrichtung und sind, in radialer Richtung gesehen, paarweise einander gegenüberliegend angeordnet, wie dies auch in Fig. 5 ersichtlich ist.

Die Stege 31 und die weiteren Stege 50 bilden Sollbruchstellen und sind dazu bestimmt, bei der Überführung von der Abdeck-Position in eine Freigabe-Position zu brechen.

Auf der Oberfläche der Aussenumhüllung 12 weist die Aussenumhüllung 12 wenigstens annähernd elliptische, sich von dem distalen Scheitelbereich des Abdeckbereichs 22 bis zum proximalen Ende der Mantelwand 24 radial nach aussen erstreckende, in Umfangsrichtung der Aussenumhüllung gleichmässig verteilte und gleich ausgebildete Wölbungen 52 zur Erleichterung der Einführung der Schutzabdeckung 10 in den Darm auf.

Ferner umfasst die Aussenumhüllung 12 eine im Innenraum angeordnete, umlaufende Stützkontur 54 zur axialen Positionierung des Kopfbereichs, wie dies in Fig. 5 und Fig. 6 ersichtlich ist. Die Stützkontur ist in der Form von radial nach innen abstehenden, im Umfangsrichtung der Aussenumhüllung 12 gleichmässig verteilten und gleich ausgebildeten Vorsprüngen 54, deren proximale Endflächen in einer rechtwinklig zur Längsachse verlaufende Stützkonturebene W liegen.

Der lichte Durchmesser D1 der Aussenumhüllung 12 proximal zur Stützkonturebene W entspricht wenigstens annähernd dem Durchmesser des Kopfbereichs.

Die Zugeinrichtung 14 umfasst ein Zugelement 56 und ein weiteres Zugelement 58, welche sich im Wesentlichen in eine rechtwinklig zur Mittelebene M verlaufende, die Längsachse L umfassende longitudinale Ebene Z und wenigstens annähernd parallel zur Längsachse L erstrecken. Das Zugelement 56 und das weitere Zugelement 58 verlaufend in einem Abstand zueinander, der dem Aussendurchmesser D2 des chirurgischen Instruments wenigstens annähernd entspricht.

Das Zugelement 56 ist über einen Vorsprungsabschnitt 56a mit dem ersten Segment 26 und das weitere Zugelement 58 ist über einen weiteren Vorsprungsabschnitt 58a mit dem zweiten Segment 28 verbunden. Der Vorsprungsabschnitt 56a und der weitere Vorsprungsabschnitt 58a verlaufen ebenfalls in die longitudinale Ebene Z.

In ihrem distalen Endbereich erstrecken sich der Vorsprungsabschnitt 56a und der weitere Vorsprungsabschnitt 58a wenigstens annähernd parallel zur Längsachse L und in einem dem lichten Durchmesser D1 entsprechenden Abstand zueinander.

In ihrem proximalen, d.h. der Aussenumhüllung abgewandten Endbereich verlaufen der Vorsprungsabschnitt 56a und der weitere Vorsprungsabschnitt 58a in einem Abstand D2 zueinander, der dem Aussendurchmesser eines Schaftbereichs des chirurgischen Instruments wenigstens annähernd entspricht. Der Vorsprungsabschnitt 56a und der weitere Vorsprungsabschnitt 58a bilden somit einen Übergangsbereich zur Anpassung des Abstands zwischen den Zugelementen 56 bzw. 58 an den Abstand, welcher dem lichten Durchmesser D1 der Aussenumhüllung 12 entspricht.

Das Zugelement 56 und das weitere Zugelement 58 sind über zwei rechtwinklig zur Längsachse L der Schutzabdeckung 10 verlaufende, beabstandet voneinander angeordnete, offene Ringe 60a bzw. 60b verbunden, welche jeweils in eine rechtwinklig zur Längsachse der Schutzabdeckung verlaufende Ebene ausgebildet sind. Die Ringe sind dazu bestimmt, den Abstand zwischen dem Zugelement 56 und dem weiteren Zugelement 58 wenigstens annähernd konstant zu halten. Der Durchmesser der Ringe 60a bzw. 60b ist derart bemessen, dass er dem Aussendurchmesser des Schaftbereichs des chirurgischen Instruments entspricht, wie dies in Fig. 7 und Fig. 8 ersichtlich ist. Das Zugelement 56 und das weitere Zugelement 58 bilden einen Aufnahmebereich 62 für das chirurgische Instrument zwischen den Ringen 60a bzw. 60b.

Die Ringen 60a bzw. 60b weisen jeweils eine Öffnung auf, die auf der gleichen Seite der durch das Zugelement 56 und das weitere Zugelement 58 definierten longitudinalen Ebene Z ausgebildet ist.

Distal zum Aufnahmebereich 62, einschliesslich in den Aufnahmebereich 62, verlaufen das Zugelement 54 und das weitere Zugelement 58 in die longitudinale Ebene Z. Proximal zum Aufnahmebereich 62 verlaufen das Zugelement 54 und das weitere Zugelement 58 nach proximal in zunehmendem Abstand von der longitudinalen Ebene Z.

Das Zugelement 54 und das weitere Zugelement 58 sind an ihren proximalen Enden über einen halbkreisförmigen Bindungsabschnitt 64 zusammen verbunden.

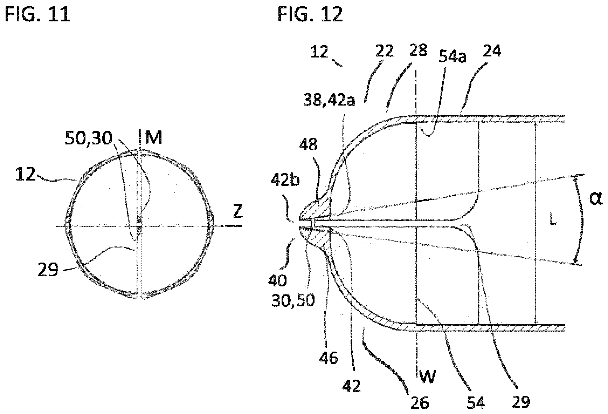

Die in Fig. 11 und Fig. 12 dargestellte, weitere Ausführungsform der Schutzabdeckung 10 ist ähnlich wie die Ausführungsform der Fig. 1 bis 10 ausgebildet. Die Bezugszeichen der Fig. 11 und Fig. 12 bezeichnen dieselben Merkmale wie in den Fig. 1 bis 10, es werden nachfolgend nur die Unterschiede beschrieben.

In Fig. 11 und Fig. 12 ist die Stützkontur 54 als einen in Umfangsrichtung verlaufenden Wulst 54a ausgebildet. Die Stützkontur 54 ist in Kontakt mit dem Kopfbereich 13 des chirurgischen Instruments über die Stützkonturfläche, welche so bemessen ist, dass der lichte Durchmesser der Aussenumhüllung L proximal zur Stützkonturebene W dem Durchmesser des Kopfbereichs entspricht.

Vorliegend ist die Oberfläche des Innenraums distal zur Stützkonturebene 54 glatt. Im Unterschied zu der in Fig. 5 dargestellten Ausführungsform sind keine Rippen vorhanden.

Weiter im Unterschied zu der in Fig. 5 dargestellten Ausführungsform erstreckt sich das Verbindungsstück 30 nur zwischen dem ersten Orientierungselement 46 und dem zweiten Orientierungselement 48. Vorliegend umfasst das Verbindungsstück 30 ein Paar Stege, d.h. zwei Stege 50, welche sich jeweils zwischen das erste Segment 26 und das zweite Segment 28 quer zum Schlitz 29 in Umfangsrichtung der Aussenumhüllung 12 erstrecken und, in radialer Richtung gesehen, einander gegenüberliegend angeordnet sind.

Es besteht somit die Möglichkeit, die Schutzabdeckung 10 mit einem weiteren Kopfbereich ebenfalls zu verwenden, der kleiner als der Durchmesser des Kopfbereichs ist. Somit passt eine Grösse der Aussenumhüllung auf wenigstens zwei Grössen des Kopfes eines chirurgischen Instruments oder von zwei verschiedenen Instrumenten.

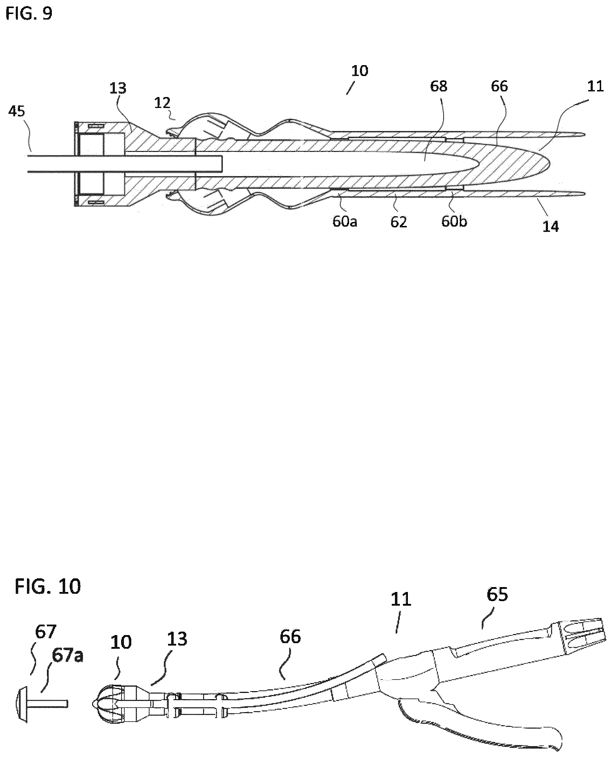

Bezugnehmend auf Fig. 10 weist das chirurgische Instrument 11, insbesondere das Klammernahtgerät oder der Zirkularstapler, eine proximale Betätigungseinrichtung 65, einen an die Betätigungseinrichtung angeschlossenen Schaftbereich 66 und den an den Schaftbereich 66 angeschlossenen Kopfbereich 13 auf. Der Schaftbereich 66 und der Kopfbereich 13 weisen ein zentral verlaufendes Lumen 68 zur Aufnahme und Führung eines intraluminalen Instruments, welches vorliegend als Dorn 45 ausgebildet ist, wie dies in Fig. 7 bis 9 dargestellt ist. Der Schaftbereich 66 und der Kopfbereich 13 definieren eine Längsachse des chirurgischen Instruments und sind wenigstens annähernd rotationsymmetrisch zur Längsachse ausgebildet, um das Einführen des chirurgischen Instruments in den Körper des Patienten zu vereinfachen.

Zudem weist das chirurgische Instrument 11 ein Gegenstück 67 auf, welches dazu bestimmt ist, bei einer Operation vor einer eigentlichen Verbindung von zwei Hohlorganteilen, insbesondere Darmhälften, mittels Klammerung, in dem von den zwei Hohlorganteilen dem chirurgischen Instrument gegenüberliegenden Hohlorganteil eingenäht zu werden. Das Gegenstück 67 ist weiter dazu bestimmt, den Dorn 45 in einen Schaft 67a des Gegenstücks 67 aufzunehmen.

Eine mögliche Verwendung der Schutzabdeckung ist nachstehend zur Veranschaulichung beschrieben. Der Vollständigkeit halber sei erwähnt, dass Abweichungen zu den unten beschriebenen Schritten, bspw. eine andere Reihenfolge, je nach Umständen auch vorstellbar sind. In einem ersten Schritt wird das chirurgische Instrument 11 in die Schutzabdeckung 10 eingeführt. Mit anderen Worten wird der Kopfbereich 13 in die Aussenumhüllung 12 und der Schaftbereich 66 in den Aufnahmebereich 62 eingeführt, sodass die Zugeinrichtung 14 nach proximal entlang die Längsachse des chirurgischen Instruments verläuft. Dabei liegt der Kopfbereich 13 in Anlage an der Stützkontur 54 an, wie in Fig.7 gezeigt. Da die Schutzabdeckung 10 und das chirurgische Instrument 11 proximal zum Aufnahmebereich 62 nach proximal in zunehmendem Abstand von der longitudinalen Ebene Z verlaufen, sind in Fig. 7 bis 8 nur der Kopfbereich und der distale Teil des Schaftbereichs sowie die Schutzabdeckung bis kurz nach dem Aufnahmebereich ersichtlich. Dabei verlaufen die Längsachse des chirurgischen Instruments und der Schutzabdeckung wenigstens annähernd aufeinander. Die Schutzabdeckung ist in der Abdeck-Position auf dem Kopfbereich 13 zentriert.

In einem zweiten Schritt werden der Kopfbereich 13 und ggf. teilweise der Schaftbereich 66 in den Körper, bspw. in den Darm, eingeführt. Ein Operateur führt den Kopfbereich einschliesslich des Dorns in den Darm ein und schiebt das chirurgische Instrument in Vorschubrichtung zur Einsatzstelle vorwärts.

Als nächstes kann der Operateur den Dorn 45 aus dem Inneren des Kopfbereichs herausfahren, wie in Fig. 8 dargestellt. Das distale Ende des Dorns 45, d.h. die Spitze des Dorns, gelangt zunächst in die Öffnung 42a und anschliessend in den Führungskanal 42 der Aussenumhüllung 12. Der Dorn wird weiter nach distal herausgefahren, bis er die Verengungsstelle erreicht und einen erhöhten Widerstand beim Ausfahren erfährt. Der Dorn 45 bleibt von der Aussenumhüllung 12 geschützt.

In einem weiteren Schritt fährt der Operateur den Dorn 45 weiter heraus und zieht die Zugeinrichtung 14 über die Zugelemente 56 bzw. 58 nach proximal. Der Druck, der dabei ausgeübt wird, ist ausreichend gross, um die Sollbruchstellen, d.h. das Verbindungsstück 30, aufzureissen. Folglich werden das das erste Segment und das erste Orientierungselement vom ersten Segment und vom zweiten Orientierungselement getrennt. Somit wird die Aussenumhüllung 12 in zwei Hälfte auseinandergetrennt, wie in Fig. 9 dargestellt. Durch Ziehen der Zugeinrichtung 14 in Richtung gegen die Vorschubrichtung können das erste Segment 26 und das zweite Segment 28 der Aussenumhüllung 12 von der Abdeck-Position in die Freigabe-Position des Kopfbereichs und des ausgefahrenen Dorns 45 überführt werden.

Die beiden Hälften können durch ein Ziehen der Zugeinrichtung entweder vor dem Klammernahtgerät oder mit diesem zusammen problemlos aus dem Darm entfernt werden. Da der Darm in radialer Richtung leicht dehnbar ist, rutschen die beiden Hälfte jeweils mittels der Zugkraft zwischen dem Kopfbereich und der Innenwand des Darms hindurch und gleiten nach proximal.

**Liste der Bezugszeichen**

| | |
|---|---|
| Schutzabdeckung | 10 |
| chirurgisches Instrument | 11 |
| Aussenumhüllung | 12 |
| Kopfbereich | 13 |
| Zugeinrichtung | 14 |
| Abdeckbereich | 22 |
| Mantelwand | 24 |
| erstes Segment | 26 |
| zweites Segment | 28 |
| Trennlinie, Schlitz | 29 |
| Verbindungsstück | 30 |
| Stege | 31 |
| Öffnung | 38 |
| Orientierungsvorrichtung | 40 |
| Führungskanal | 42 |
| Proximale und distale Führungskanalöffnung | 42a bzw. 42b |
| Dorn | 45 |
| erstes Orientierungselement | 46 |
| zweites Orientierungselement | 48 |
| weitere Stege | 50 |
| Wölbungen | 52 |
| Stützkontur | 54 |
| Wulst | 54a |
| Zugelement | 56 |
| Vorsprungsabschnitt | 56a |
| weiteres Zugelement | 58 |
| weiterer Vorsprungsabschnitt | 58a |
| Ringe | 60a bzw. 60b |
| Aufnahmebereich | 62 |
| Bindungsabschnitt | 64 |
| Betätigungseinrichtung | 65 |
| Schaftbereich | 66 |
| Gegenstück | 67 |
| Lumen | 68 |
| Längsachse | L |
| Mittelebene | M |
| Longitudinalebene | Z |
| Stützkonturebene | W |

## Patentansprüche

1. Schutzabdeckung (10) für ein chirurgisches Instrument, insbesondere für ein Klammernahtgerät, umfassend eine einen Innenraum begrenzende Aussenumhüllung (12) zur wenigstens teilweisen Abdeckung eines Kopfbereichs des in die Schutzabdeckung (10) entlang deren Längsachse L eingeführten chirurgischen Instruments, und eine Zugeinrichtung (14), welche mit der Aussenumhüllung (12) kraftübertragend verbunden ist und ein Zugelement (56) umfasst, wobei die Aussenumhüllung (12) eine Trennlinie (29) aufweist, welcher die Aussenumhüllung (12) in wenigstens ein erstes Segment (26) und ein zweites Segment (28) teilt, das erste Segment (26) und das zweite Segment (28) durch ein Verbindungsstück (30) miteinander verbunden sind und mit Hilfe der Zugeinrichtung (14) von einer Abdeck-Position in eine Freigabe-Position des Kopfbereichs überführbar sind, **dadurch gekennzeichnet, dass** die Aussenumhüllung (12) eine in ihrem distalen Scheitelbereich angeordnete, auf die Längsachse zentrierte Orientierungsvorrichtung (40) umfasst, welche als eine Verdickung des Scheitelbereichs ausgebildet ist.

2. Schutzabdeckung (10) nach Anspruch 1, **dadurch gekennzeichnet, dass** die Orientierungsvorrichtung (40) dazu bestimmt ist, den Ort des Durchstechens eines Dorns in ein Organ zu ertasten, welcher Dorn vom Kopfbereich des in die Schutzabdeckung (10) eingeführten chirurgischen Instruments axial absteht.

3. Schutzabdeckung (10) nach Anspruch 1 oder 2, **dadurch gekennzeichnet, dass** das Stege aufweisende Verbindungsstück (30) eine Sollbruchstelle bildet, welche dazu bestimmt ist, bei der Überführung von der Abdeck-Position in die Freigabe-Position zu brechen.

4. Schutzabdeckung (10) nach einem der Ansprüche 1 bis 3, **dadurch gekennzeichnet, dass** die Orientierungsvorrichtung (40) auf der dem Innenraum abgewandten Seite der Aussenumhüllung (12) absteht.

5. Schutzabdeckung (10) nach einem der Ansprüche 1 bis 4, **dadurch gekennzeichnet, dass** die Orientierungsvorrichtung (40) als eine sich nach distal verjüngende Verdickung des Scheitelbereichs ausgebildet ist.

6. Schutzabdeckung (10) nach einem der Ansprüche 1 bis 5, **dadurch gekennzeichnet, dass** die Orientierungsvorrichtung gewölbt ausgebildet ist.

7. Schutzabdeckung (10) nach einem der Ansprüche 1 bis 4, **dadurch gekennzeichnet, dass** die Orientierungsvorrichtung (40) als eine sich nach distal entlang der Längsachse prismenförmig oder zylinderförmig erstreckende Verdickung des Scheitelbereichs ausgebildet ist.

8. Schutzabdeckung (10) nach einem der Ansprüche 1 bis 7, **dadurch gekennzeichnet, dass** die Trennlinie (29) die Orientierungsvorrichtung (40) in ein erstes Orientierungselement (46) und ein zweites Orientierungselement (48) teilt, wobei das erste Orientierungselement (46) mit dem ersten Segment (26) und das zweite Orientierungselement (48) mit dem zweiten Segment (28) verbunden sind.

9. Schutzabdeckung (10) nach einem der Ansprüche 1 bis 8, **dadurch gekennzeichnet, dass** die Zugeinrichtung (14) ein weiteres Zugelement (58) umfasst, wobei vorzugsweise das Zugelement (56) und das weitere Zugelement (58) in eine longitudinale Ebene Z verlaufen, welche die Längsachse L der Schutzabdeckung (10) enthält.

10. Schutzabdeckung (10) nach einem der Ansprüche 1 bis 9, **dadurch gekennzeichnet, dass** die Trennlinie (29) in eine Mittelebene M verläuft, welche die Längsachse L der Schutzabdeckung (10) enthält.

11. Schutzabdeckung (10) nach einem der Ansprüche 1 bis 10, **dadurch gekennzeichnet, dass** die Aussenumhüllung (12) rotationssymmetrisch um die Längsachse L ausgebildet ist.

12. Schutzabdeckung (10) nach einem der Ansprüche 1 bis 11, **dadurch gekennzeichnet, dass** die Oberfläche der Aussenumhüllung (12) zungenförmige, sich von dem distalen zum proximalen Ende der Aussenumhüllung erstreckende, in Umfangsrichtung der Aussenumhüllung gleichmässig verteilte und gleich ausgebildete Wölbungen aufweist.

13. Schutzabdeckung (10) nach einem der Ansprüche 1 bis 12, **gekennzeichnet durch** eine einstückige Ausbildung.

14. Schutzabdeckung (10) nach einem der Ansprüche 1 bis 13, **dadurch gekennzeichnet, dass** die Aussenumhüllung (12) eine in deren Innenraum angeordneten, vorzugsweise umlaufende Stützkontur (54) zur axialen Positionierung des Kopfbereichs in die Aussenumhüllung (12) umfasst.

15. Schutzabdeckung (10) nach Anspruch 14, **dadurch gekennzeichnet, dass** die Stützkontur (54) als radial nach innen abstehende, im Umfangsrichtung der Aussenumhüllung verteilte Vorsprünge umfasst, deren proximale Endflächen in einer rechtwinklig zur Längsachse L verlaufende Stützkonturebene W liegen.

## Claims

1. Protective cover (10) for a surgical instrument, in particular for a stapler, comprising an outer sheath (12) delimiting an inner space for at least partially covering a head region of the surgical instrument inserted into the protective cover (10) along the longitudinal axis L thereof, and a pulling device (14) which is connected to the outer sheath (12) in a force-transmitting manner and comprises a pulling element (56), the outer sheath (12) having a dividing line (29) which divides the outer sheath (12) into at least a first segment (26) and a second segment (28), the first segment (26) and the second segment (28) are connected to one another by a connecting piece (30) and can be transferred from a covering position into a release position of the head region with the aid of the pulling device (14), **characterized in that** the outer sheath (12) comprises an orientation device (40) which is arranged in its distal apex region, is centered on the longitudinal axis and is designed as a thickening of the apex region.

2. Protective cover (10) according to claim 1, **characterized in that** the orientation device (40) is intended to sense the location of the piercing of a mandrel into an organ, which mandrel projects axially from the head region of the surgical instrument inserted into the protective cover (10).

3. Protective cover (10) according to claim 1 or 2, **characterized in that** the connecting piece (30) having webs forms a predetermined breaking point which is intended to break during the transfer from the covering position to the release position.

4. Protective cover (10) according to one of claims 1 to 3, **characterized in that** the orientation device (40) protrudes on the side of the outer sheath (12) facing away from the interior.

5. Protective cover (10) according to one of claims 1 to 4, **characterized in that** the orientation device (40) is designed as a distally tapering thickening of the apex region.

6. Protective cover (10) according to one of claims 1 to 5, **characterized in that** the orientation device is of curved design.

7. Protective cover (10) according to one of claims 1 to 4, **characterized in that** the orientation device (40) is designed as a thickening of the apex region extending distally along the longitudinal axis in a prismatic or cylindrical shape.

8. Protective cover (10) according to one of claims 1 to 7, **characterized in that** the dividing line (29) divides the orientation device (40) into a first orientation element (46) and a second orientation element (48), the first orientation element (46) being connected to the first segment (26) and the second orientation element (48) being connected to the second segment (28).

9. Protective cover (10) according to one of claims 1 to 8, **characterized in that** the pulling device (14) comprises a further pulling element (58), wherein preferably the pulling element (56) and the further pulling element (58) extend in a longitudinal plane Z, which contains the longitudinal axis L of the protective cover (10).

10. Protective cover (10) according to one of claims 1 to 9, **characterized in that** the dividing line (29) runs in a central plane M, which contains the longitudinal axis L of the protective cover (10).

11. Protective cover (10) according to one of claims 1 to 10, **characterized in that** the outer sheath (12) is designed to be rotationally symmetrical about the longitudinal axis L.

12. Protective cover (10) according to one of claims 1 to 11, **characterized in that** the surface of the outer sheath (12) has tongue-shaped bulges extending from the distal to the proximal end of the outer sheath, evenly distributed and uniformly formed in the circumferential direction of the outer sheath.

13. Protective cover (10) according to one of claims 1 to 12, **characterized by** a one-piece design.

14. Protective cover (10) according to one of claims 1 to 13, **characterized in that** the outer sheath (12) comprises a preferably circumferential support contour (54) arranged in its interior for axial positioning of the head region in the outer sheath (12).

15. Protective cover (10) according to claim 14, **characterized in that** the support contour (54) comprises projections which project radially inwards and are distributed in the circumferential direction of the outer sheath and whose proximal end faces lie in a support contour plane W extending at right angles to the longitudinal axis L.

## Revendications

1. Housse de protection (10) pour un instrument chirurgical, en particulier pour une agrafeuse, comprenant une gaine extérieure (12) délimitant un espace intérieur pour recouvrir au moins partiellement une région de tête de l'instrument chirurgical inséré dans la housse de protection (10) le long de son axe longitudinal L, et un dispositif de traction (14) qui est relié à la gaine extérieure (12) de manière à transmettre une force et comprend un élément de traction (56), la gaine extérieure (12) présentant une ligne de séparation (29) qui divise la gaine extérieure (12) en au moins un premier segment (26) et un deuxième segment (28), le premier segment (26) et le deuxième segment (28) étant reliés l'un à l'autre par une pièce de liaison (30) et peuvent être transférés d'une position de recouvrement à une position de libération de la région de tête à l'aide du dispositif de traction (14), **caractérisée en ce que** la gaine extérieure (12) comprend un dispositif d'orientation (40) qui est disposé dans sa région apicale distale, est centré sur l'axe longitudinal et est conçu comme un épaississement de la région apicale.

2. Housse de protection (10) selon la revendication 1, **caractérisée en ce que** le dispositif d'orientation (40) est destiné à détecter l'emplacement de la perforation d'un mandrin dans un organe, lequel mandrin fait saillie axialement à partir de la région de tête de l'instrument chirurgical inséré dans la housse de protection (10).

3. Housse de protection (10) selon la revendication 1 ou 2, **caractérisée en ce que** la pièce de liaison (30) comportant des nervures forme un point de rupture prédéterminé qui est destiné à se rompre lors du transfert de la position de recouvrement à la position de libération.

4. Housse de protection (10) selon l'une des revendications 1 à 3, **caractérisée en ce que** le dispositif d'orientation (40) fait saillie sur le côté de la gaine extérieure (12) opposé à l'intérieur.

5. Housse de protection (10) selon l'une des revendications 1 à 4, **caractérisée en ce que** le dispositif d'orientation (40) est conçu comme un épaississement effilé distalement de la région apicale.

6. Housse de protection (10) selon l'une des revendications 1 à 5, **caractérisée en ce que** le dispositif d'orientation est de conception courbée.

7. Housse de protection (10) selon l'une des revendications 1 à 4, **caractérisée en ce que** le dispositif d'orientation (40) est conçu comme un épaississement de la région apicale s'étendant distalement le long de l'axe longitudinal sous une forme prismatique ou cylindrique.

8. Housse de protection (10) selon l'une des revendications 1 à 7, **caractérisée en ce que** la ligne de séparation (29) divise le dispositif d'orientation (40) en un premier élément d'orientation (46) et un deuxième élément d'orientation (48), le premier élément d'orientation (46) étant relié au premier segment (26) et le deuxième élément d'orientation (48) étant relié au deuxième segment (28).

9. Housse de protection (10) selon l'une des revendications 1 à 8, **caractérisée en ce que** le dispositif de traction (14) comprend un autre élément de traction (58), de préférence, l'élément de traction (56) et l'autre élément de traction (58) s'étendant dans un plan longitudinal Z qui contient l'axe longitudinal L de la housse de protection (10).

10. Housse de protection (10) selon l'une des revendications 1 à 9, **caractérisée en ce que** la ligne de séparation (29) s'étend dans un plan central M qui contient l'axe longitudinal L de la housse de protection (10).

11. Housse de protection (10) selon l'une des revendications 1 à 10, **caractérisée en ce que** la gaine extérieure (12) est conçue pour être symétrique en rotation autour de l'axe longitudinal L.

12. Housse de protection (10) selon l'une des revendications 1 à 11, **caractérisée en ce que** la surface de la gaine extérieure (12) présente des renflements en forme de languettes s'étendant de l'extrémité distale à l'extrémité proximale de la gaine extérieure, répartis de manière régulière et formés uniformément dans la direction circonférentielle de la gaine extérieure.

13. Housse de protection (10) selon l'une des revendications 1 à 12, **caractérisée par** une conception monobloc.

14. Housse de protection (10) selon l'une des revendications 1 à 13, **caractérisée en ce que** la gaine extérieure (12) comprend un contour de support (54) de préférence circonférentiel disposé à l'intérieur de celle-ci pour le positionnement axial de la région de tête dans la gaine extérieure (12).

15. Housse de protection (10) selon la revendication 14, **caractérisée en ce que** le contour de support (54) comprend des saillies qui font saillie radialement vers l'intérieur et sont réparties dans la direction circonférentielle de la gaine extérieure et dont les faces d'extrémité proximales se trouvent dans un plan de contour de support W s'étendant perpendiculairement à l'axe longitudinal L.
